# EUROPÄISCHE PATENTSCHRIFT

(11) **EP 3 049 136 B1**
(45) Veröffentlichungstag und Bekanntmachung des Hinweises auf die Patenterteilung: **17.07.2019**
(21) Anmeldenummer: 14795539.7
(22) Anmeldetag: 26.09.2014
(51) Int. Cl.: A61M 13/00, A61M 16/16, A61M 16/20

(54) **DRUCKERHALTENDE RAUCHGASABSAUGUNG IN EINEM INSUFFLATOR**
PRESSURE-MAINTAINING SMOKE EVACUATION IN AN INSUFFLATOR
ASPIRATION D'EFFLUENTS GAZEUX AVEC MAINTIEN DE LA PRESSION DANS UN INSUFFLATEUR

(30) Priorität: 27.09.2013 DE 102013016063
(43) Veröffentlichungstag der Anmeldung: 03.08.2016
(60) Teilanmeldung: 19000224.6
(73) Patentinhaber: W.O.M. World of Medicine GmbH, 10587 Berlin (DE)
(72) Erfinder: KÖTH, Yves, 12683 Berlin (DE); STEGEMANN, Sebastian, 12105 Berlin (DE)
(74) Vertreter: Jungblut & Seuss
(86) Internationale Anmeldenummer: PCT/DE2014/000482
(87) Internationale Veröffentlichungsnummer: WO 2015/043570

(56) Entgegenhaltungen:
- WO-A1-2004/009167
- WO-A1-2011/041387
- WO-A1-2012/012379
- DE-A1- 4 219 859
- DE-C1- 3 922 746
- US-A- 4 735 603
- US-A- 4 836 187
- US-A- 4 971 034

## Beschreibung

Die vorliegende Erfindung betrifft einen Insufflator mit einer Rauchgasabsaugung welcher so ausgestaltet ist, dass der abdominelle Druck während der Absaugung weitestgehend konstant bleibt. Die Priorität der deutschen Anmeldung DE102013016063.4 (Anmeldetag 27.9.2014) wird in Anspruch genommen.

### Hintergrund und Stand der Technik

Im Bereich der minimal-invasiven Chirurgie, zum Beispiel der diagnostischen oder therapeutischen Laparoskopie wird im Abdomen mittels eingeleiteter Gase (z. B. medizinisches Kohlendioxid (CO₂)) ein Überdruck erzeugt, welcher das Abdomen aufdehnt, damit ausreichend Platz für die visuelle Inspektion bzw. den therapeutischen Eingriff besteht. Hierzu werden sogenannte Insufflatoren verwendet, welche mittels eines geregelten Gasflusses einen kontrollierten Druck im Abdomen aufbauen. Über eine Reihe von Sicherheitsvorrichtungen wird sichergestellt, dass sich der abdominelle Druck in Grenzen hält, so dass kein Gewebe geschädigt wird.

Im Rahmen der minimal-invasiven Chirurgie werden häufig elektrochirurgische Geräte oder auch Laser verwendet, deren Anwendung Rauch im Abdomen erzeugt. Dieser Rauch behindert nicht nur die Sicht, sondern enthält auch Schadstoffe (in Form von Gasen, Tröpfchen und/oder Partikel), so dass dieser aus dem Abdomen entfernt werden muss. Eine Lösung dieses Problems besteht darin, einen einfachen Auslass zu schaffen, über den die Rauchgase den Körper verlassen. Eine Realisierungsmöglichkeit hierfür wird in der US 6592543 gezeigt. Die Absaugung wird dabei durch den Operateur manuell durch das öffnen des Ventils am Trokar gesteuert. Allerdings werden durch heutige Systeme häufig größere Rauchmengen erzeugt, als zum Anmeldezeitpunkt der US 6592543, so dass die dort vorgestellte Lösung heute nicht mehr anwendbar ist.

Eine Weiterentwicklung derart passiver Systeme besteht durch die Bereitstellung einer Absaugvorrichtung, die das Gas im Abdomen inklusive der Rauchpartikel aktiv absaugen kann. Ein derartiges System wird beispielsweise in der US 5199944 beschrieben. Dabei besteht das Problem, dass bei zu starker Absaugung der Rauchgase der Druck im Abdomen sinkt, so dass das Abdomen in sich zusammenfallen kann. Der behandelnde Arzt muss dann mit der Weiterführung der Operation solange warten, bis sich im Abdomen wieder ausreichend Druck aufgebaut hat. Es ist ohne weiteres einsichtig, dass diese Vorgehensweise enorme Nachteile mit sich bringt. Anderseits ist bei zu schwacher Absaugung diese nicht effektiv genug, so dass der Operateur durch die schlechte Sicht behindert wird und die Operation verlängert wird. Eine Alternative zu dieser Vorgehensweise besteht darin, das abgesaugte Gas dem Patienten wieder zuzuführen (Zirkulation). Ein derartiges System wird in dem Patent US 4735603 beschrieben. Auch in dieser Ausführungsform besteht das Problem, das bei zu hoher Absaugrate der abdominelle Druck sinkt.

Bei einer hohen Absaugleistung steigt der Systemdruck auf der Zuführleitung stark an. Je nachdem, wie eng das angeschlossene Instrument ist, kann der Druck stärker oder schwächer ansteigen. In jedem Fall wird ab einer bestimmten Druckgrenze das Sicherheitsventil innerhalb der Zuführleitung öffnen und schon damit zu einem Druckabfall führen. Weiterhin kann der Insufflator kein zusätzliches Kohlendioxid mehr zuführen, um wahrscheinliche vorhandene Leckagen auszugleichen.

Sowohl bei einer Absaugung mit als auch ohne Zirkulation ergibt sich das Problem, dass die Absaugleistung an die aktuellen Bedingungen der Operation angepasst werden müssen. Wie beschrieben, spielen hier die verwendeten Instrumente, aber auch die aktuellen Leckraten am Abdomen eine entscheidende Rolle.

Stand der Technik ist weiterhin, dass dem Operateur am Gerät verschiedene Stufen für die Absaugleistung angeboten werden (beispielsweise: Niedrig / Mittel / Hoch). Der Operateur hat damit die Verantwortung, die optimale Absaugstufe festzulegen. Sollte die Absaugleistung zu hoch eingestellt sein, kann der Insufflator den Bauchdruck nicht aufrecht erhalten. Dies bemerkt der Operateur üblicherweise erst, wenn der Druck bereits deutlich zurückgegangen ist. Weiterhin ist es für den Operateur schwierig, im Vorhinein zu erkennen, dass auch eine höhere Absaugleistung möglich und auch nötig ist. Möglicherweise wählt der Operateur dann erst gar nicht die höhere Absaugleistung aus, um den Druckabfall nicht zu riskieren.

Weiterer Stand der Technik wird gebildet durch die folgenden Dokumente:
WO 2011/041387 A1 beschreibt ein Gaszuleitungssystem für laparoskopische Anwendungen. Hierbei kann Gas aus einem Behandlungsraum abgesaugt werden. Das Gas wird dem Körper anschließend wieder zugeführt, so dass keine Druckreduktion möglich ist.
DE 4219859 A1 beschreibt einen Insufflator mit der Möglichkeit einer Gaszuleitung und einer Gasableitung. Gaszu- und -ableitung wird über eine einzige Leitung vorgenommen.
WO 2004/009167 A1 beschreibt eine Vorrichtung zur Insufflation einer Körperhöhle einen Schlauch, welcher seinerseits eine Gasableitung enthält, die zu einer Messvorrichtung führt. Die Messvorrichtung kann insbesondere prüfen, ob sich im Abdomen einen zündfähiges Gasgemisch angesammelt hat.
WO 2012/012379 A1 beschreibt ein Gasmanagementsystem für die Laparoskopie, bei dem Gasflüsse auf der Basis von In-vivo-Sensoren gesteuert werden.

Es besteht daher Bedarf an einer Vorrichtung für die minimal-invasive Chirurgie, insbesondere die Laparoskopie, die es erlaubt, die höchstmögliche Absaugrate für Rauchgase aus dem Behandlungsraum (insbesondere dem Abdomen) zu bestimmen und einzustellen, so dass kein signifikanter Druckabfall im genannten Volumen stattfindet. Dabei sollen die im Markt üblichen Trokare verwendet werden können. Die Lösung des Problems wird durch die nachfolgend beschriebene erfindungsgemäße Vorrichtung gewährleistet.

### Lösung der Aufgabe

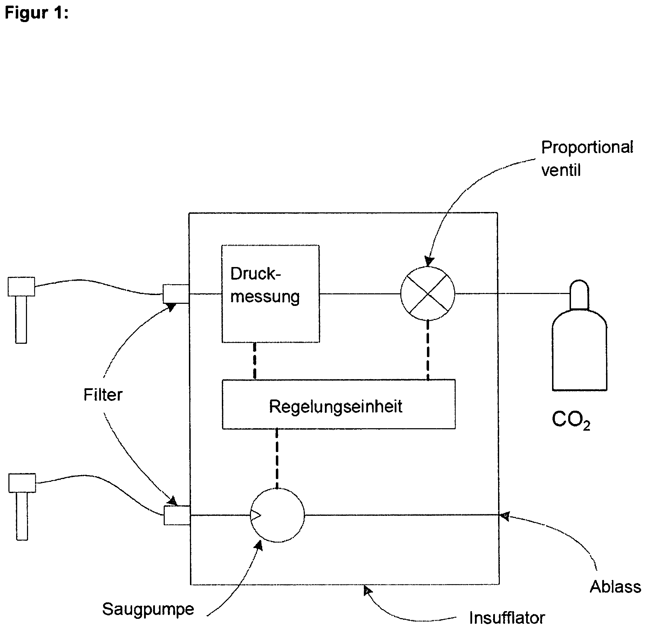

Die Lösung der Aufgabe erfolgt durch den Gegenstand des Anspruchs 1. Bevorzugte Ausführungsformen werden in den Unteransprüchen definiert. Ein erfindungsgemäßer Insufflator enthält nämlich eine Absaugvorrichtung mit regelbarer Absaugleistung, beispielsweise eine integrierte Absaugpumpe. Eine derartige Pumpe ist beispielhaft in der Figur 1, die einen erfindungsgemässen Insufflator zeigt, dargestellt. Der Insufflator enthält zunächst einen üblichen Anschluss an eine Druckgasflasche mit einem Gas, welches für medizinische Zwecke geeignet ist, z. B. Kohlendioxid. Das Gas wird zunächst über ein Proportionalventil und einem sterilem Schlauch einem Trokar zugeführt. Innerhalb des Insufflators wird sowohl der Druck, als auch der Gasfluss (Flow) der Leitung gemessen. Ein Filter wird noch eingeführt, der Partikel zurückhält. Ein zweiter Trokar wird an eine in den Insufflator integrierte Saugpumpe angeschlossen. Dabei wird wiederum ein Filter zwischengeschaltet, der Partikel, Tröpfchen und/oder giftige Gase absorbieren kann. Das abgepumpte Insufflationsgas kann dann in die Atmosphäre entlassen werden. Der Insufflator wird über eine Schalteinheit (Control Circuit) gesteuert. Dieser regelt insbesondere die Leistung der Saugpumpe auf elektronischem Wege.

Die vorliegende Erfindung besteht daher aus einem Insufflator für die minimal-invasive Chirurgie, enthaltend
a) Gasanschluss mit Proportionalventil,
b) einen ersten Trokar,
c) eine Gaszuführleitung für Gas aus dem Gasanschluss mit Druckmessung, Filter und Anschluss an den ersten Trokar,
d) eine Absaugvorrichtung mit regelbarer Absaugleistung,
e) einen zweiten Trokar mit Absaugschlauch und Filter, angeschlossen an die Absaugvorrichtung mit regelbarer Absaugleistung, und
f) eine elektronische Regelungseinheit,
wobei die elektronische Regelungseinheit die Absaugvorrichtung in Abhängigkeit vom Druck in der Zuführleitung regelt,
wobei die elektronische Regeleinheit zur Steuerung der Absaugvorrichtung so konfiguriert ist,
dass die Absaugrate verkleinert wird, wenn der Druck in der Zuführleitung einen Schwellwert, der zwischen 8,0 kPa (60 mmHg) und 10,7 kPa (80 mmHg) liegt, überschreitet und
dass die Absaugrate erhöht wird, wenn der Druck in der Zuführleitung einen Schwellwert, der zwischen 0 und 6,7 kPa (50 mmHg) liegt, unterschreitet. In speziellen Ausführungsformen der Erfindung sind die Schwellwerte am Insufflator einstellbar.

Die maximale Flussrate des Insufflators wird maßgeblich von den angeschlossenen Instrumenten am Insufflationsschlauch bestimmt. Bei Verwendung eines kleinen bzw. engen Trokars in Kombination mit einem großen Instrument kann die maximale Insufflationsleistung 4 - 6 l/min oder weniger betragen. Bei Verwendung von großen Trokaren kann die maximale Insufflationsleistung bei 15 - 20 l/min oder sogar darüber hinaus liegen.

Der Insufflator regelt das nachzufüllende Volumen nach dem jeweiligen Bedarf. Selbstverständlich wird bereits aus Sicherheitsgründen ein maximaler Druck im Zuführschlauch definiert, der nicht überschritten werden darf. Dieser maximale Druck limitiert den maximalen Gasfluss.

Der Bedarf zum Nachfüllen von Kohlendioxid wird durch permanente sowie kurzzeitige Leckagen erzeugt. Die im Patienten eingesetzen Trokare weisen bauartbedingt minimale Leckagen auf. Weiterhin kann die aktive Rauchgasabsaugung gewissermaßen als eine permanente Leckage angesehen werden, die durch den Insufflator ausgeglichen werden muss.

Wenn beispielsweise intraoperativ eine Absaugung mit einer Flussrate von 4 l/min. vorhanden ist und eine systembedingte Leckage von 3 l/min., dann muss der Insufflator einen Flow von ∼ 7 l/min im Zufluss einstellen. Der Insufflator wird zum Erreichen des Zielflow den Druck im Zuführschlauch solange erhöhen, bis sich entweder der Flow einstellt, oder der maximal zulässige Druck erreicht wurde. Wird der maximale Druck erreicht bevor der Zielflow erreicht wurde, kann die Leckage durch den Insufflator nicht ausgeglichen werden. Sollte dieser Zustand länger anhalten, würde der abdominale Druck fallen. Aus diesem Grund muss der Insufflator in diesem Fall die Absaugleistung verringern.

Wenn jedoch ausreichend Kapazität im Schlauch bzw. Instrument vorhanden ist, kann der geforderte Flow von 7 l/min. eingestellt werden, ohne dass der maximale Druck erreicht wird. In diesem Fall kann die Absaugleistung sogar noch erhöht werden.

Der erreichte Druck im Zuführschlauch im Verhältnis zum maximalen Druck während der Insufflation ist also ein Maß, welches die noch verfügbare Kapazität im Schlauch bzw. Instrument anzeigt.

Die Regelung der Leistung kann mittels des oben beschriebenen Verhältnisses aus erreichtem Treiberdruck zu maximalem Treiberdruck geregelt werden. Dabei kann direkt die Eingangsspannung der Pumpe variiert werden.

Im Ergebnis wird die Leistung der Rauchgasabsaugung auf die aktuellen Bedingungen der Insufflationsleistung angepasst bzw. optimiert. Damit wird die maximale Absaugleistung eingestellt, ohne dass der Patientendruck sich ändern würde.

Der maximale Treiberdruck der erfindungsgemäßen Vorrichtung liegt üblicherweise bei 10,7 kPa (80 mmHg).

### Alternative Ausführungsformen und nicht erfindungsgemäße Beispiele

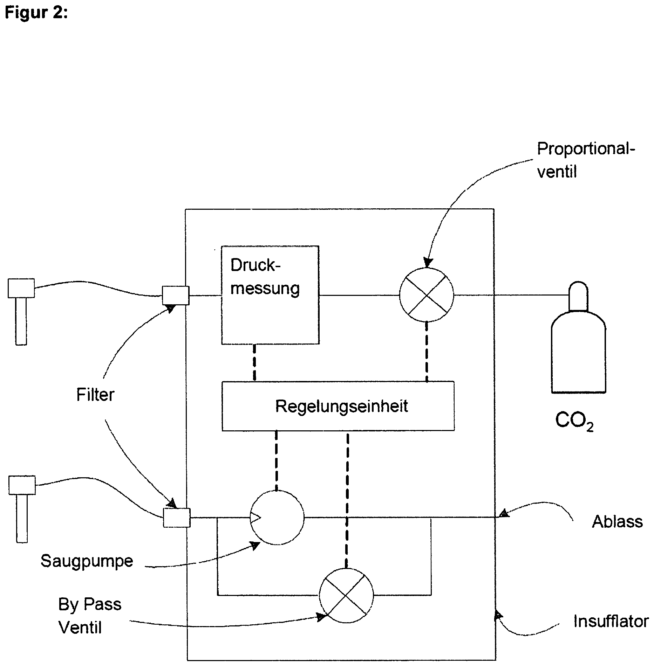

Der Fachmann auf dem Gebiet, angeregt von der vorliegenden Beschreibung, kann den Grundgedanken der Erfindung auch mit anderen Mitteln realisieren, wobei allerdings die Erfindung ausschließlich von dem Gegenstand des Anspruchs 1 definiert wird. So kann beispielsweise die Saugpumpe über einen ByPass-Ventil gesteuert werden (s. Figur 2, die einen weiteren erfindungsgemäßen Insufflator zeigt). Hierzu kann beispielsweise die Pumpe auf eine bestimmte Leistung eingestellt werden, welche weitestgehend konstant ist, und die Regelung der Leistung erfolgt dann über das ByPass-Ventil.

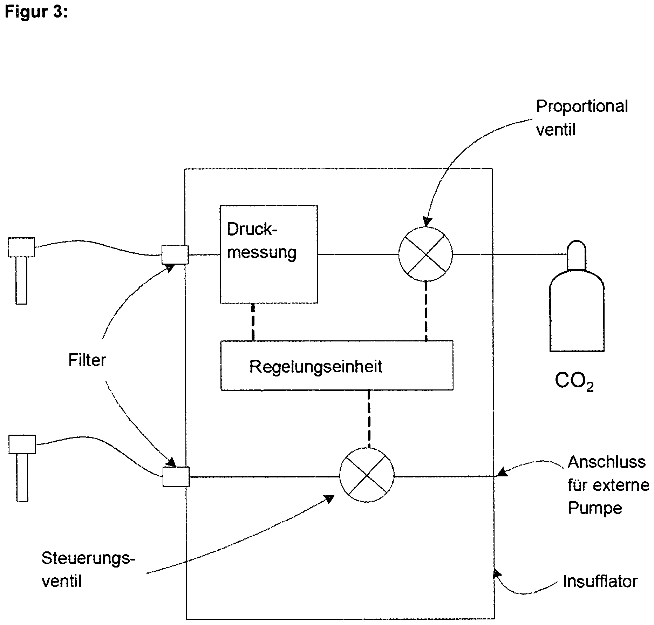

In einem nicht erfindungsgemäßen Beispiel kann auch ein Steuerungsventil direkt in die Absaugleitung positioniert werden (s. Figur 3, die einen nicht erfindungsgemäßen Insufflator zeigt). Auf diese Weise könnten sogar externe Pumpen verwendet werden, beispielsweise die im OP vorhandene Wandsaugung. Das Kontrollsystem des Insufflators regelt dann die Absaugleistung über das dargestellte Steuerungsventil.

In weiteren Ausführungsformen der Erfindung kann die Gaszuführleitung vom Insufflator zum Patienten auch heizbar ausgestaltet sein, beispielsweise über einen Schlauch, wie er in der WO2014/111084 beschrieben wird. Alternativ kann die Gaszuführleitung vom Insufflator zum Patienten nicht nur heizbar sondern auch zur Befeuchtung des Gases eingerichtet sein, beispielsweise über einen Schlauch, wie er in der WO2014/111083 beschrieben wird.

## Patentansprüche

1. Insufflator für die minimal-invasive Chirurgie, enthaltend
a) Gasanschluss mit Proportionalventil,
b) einen ersten Trokar,
c) eine Gaszuführleitung für Gas aus dem Gasanschluss mit Druckmessung, Filter und Anschluss an den ersten Trokar,
d) eine Absaugvorrichtung mit regelbarer Absaugleistung,
e) einen zweiten Trokar mit Absaugschlauch und Filter, angeschlossen an die Absaugvorrichtung mit regelbarer Absaugleistung und
f) eine elektronische Regelungseinheit,
wobei die elektronische Regelungseinheit die Absaugvorrichtung in Abhängigkeit vom Druck in der Zuführleitung regelt,
**dadurch gekennzeichnet, dass** die elektronische Regeleinheit zur Steuerung der Absaugvorrichtung so konfiguriert ist,
dass die Absaugrate verkleinert wird, wenn der Druck in der Zuführleitung einen Schwellwert, der zwischen 8,0 kPa (60 mmHg) und 10,7 kPa (80 mmHg) liegt, überschreitet und
dass die Absaugrate erhöht wird, wenn der Druck in der Zuführleitung einen Schwellwert, der zwischen 0 und 6,7 kPa (50 mmHg) liegt, unterschreitet.

2. Insufflator gemäß Anspruch 1, **dadurch gekennzeichnet, dass** die Absaugvorrichtung mit regelbarer Absaugleistung
aus einer regelbaren Absaugpumpe
oder
aus einer Absaugpumpe mit einem regelbaren By-Pass Ventil
oder
aus einer Absaugpumpe mit einem zusätzlichen regelbaren Proportionalventil besteht.

3. Insufflator gemäß mindestens einem der Ansprüche 1-2, **gekennzeichnet durch** eine zwischen 0 und 50 l/min regelbare Insufflationsleistung.

4. Insufflator gemäß Anspruch 3, **gekennzeichnet durch** eine zwischen 0 und 20 l/min regelbare Absaugleistung.

5. Insufflator gemäß Anspruch 4, **gekennzeichnet durch** eine zwischen 0 und 10 l/min regelbare Absaugleistung.

6. Insufflator gemäß mindestens einem der Ansprüche 1-5, **gekennzeichnet durch** eine Gasheizvorrichtung in der Gaszuführleitung.

## Claims

1. An insufflator for minimally invasive surgeries, comprising
a) a gas connection with a proportional valve,
b) a first trocar,
c) a gas supply line for gas from the gas connection with pressure measurement, filter, and connection to the first trocar,
d) a suction device with controllable suction capacity,
e) a second trocar with suction hose and filter, connected to the suction device with controllable suction capacity, and
f) an electronic control unit,
the electronic control unit controlling the suction device in dependence of the pressure in the supply line,
**characterized by** that the electronic control unit for controlling the suction device is configured such that the suction rate is decreased, when the pressure in the supply line exceeds a threshold value being between 8.0 kPa (60 mm Hg) and 10.7 kPa (80 mm Hg), and
that the suction rate is increased, when the pressure in the supply line falls below a threshold value being between 0 and 6.7 kPa (50 mm Hg).

2. The insufflator of claim 1, **characterized by** that the suction device with controllable suction capacity consists of
a controllable suction pump or
a suction pump with a controllable by-pass valve or
a suction pump with an additional controllable proportional valve.

3. The insufflator of at least one of claims 1 to 2, **characterized by** an insufflation capacity controllable between 0 and 50 l/min.

4. The insufflator of claim 3, **characterized by** a suction capacity controllable between 0 and 20 l/min.

5. The insufflator of claim 4, **characterized by** a suction capacity controllable between 0 and 10 l/min.

6. The insufflator of at least one of claims 1 to 5, **characterized by** a gas heating device in the gas supply line.

## Revendications

1. Insufflateur pour la chirurgie mini-invasive, comprenant
a) une prise gaz avec une vanne proportionnelle,
b) un premier trocart,
c) une conduite d'alimentation à partir de la prise gaz avec mesurage de pression, filtre et raccordement au premier trocart,
d) un dispositif d'aspiration avec puissance d'aspiration réglable,
e) un deuxième trocart avec tuyau d'aspiration et filtre, raccordé au dispositif d'aspiration avec puissance d'aspiration réglable, et
f) une unité de réglage électronique,
l'unité de réglage électronique réglant le dispositif d'aspiration en fonction de la pression dans la conduite d'alimentation,
**caractérisé en ce que** l'unité de réglage électronique pour régler le dispositif d'aspiration est configurée de manière à réduire le débit d'aspiration, si la pression dans la conduite d'alimentation est supérieure à un seuil entre 8,0 kPa (60 mm Hg) et 10,7 kPa (80 mm Hg), et
à augmenter le débit d'aspiration, si la pression dans la conduite d'alimentation est inférieure à un seuil entre 0 et 6,7 kPa (50 mm Hg).

2. Insufflateur selon la revendication 1, **caractérisé en ce que** le dispositif d'aspiration avec puissance d'aspiration réglable consiste en une pompe d'aspiration réglable ou
une pompe d'aspiration avec une vanne de by-pass réglable ou
une pompe d'aspiration avec une vanne proportionnelle réglable additionnelle.

3. Insufflateur selon au moins une des revendications 1 à 2, caractérisé en une puissance d'insufflation réglable entre 0 et 50 l/min.

4. Insufflateur selon la revendication 3, caractérisé en une puissance d'aspiration réglable entre 0 et 20 l/min.

5. Insufflateur selon la revendication 4, caractérisé en une puissance d'aspiration réglable entre 0 et 10 1/min.

6. Insufflateur selon au moins une des revendications 1 à 5, caractérisé en un dispositif de chauffage de gaz dans la conduite d'alimentation de gaz.
